# FASCICULE DE BREVET EUROPEEN

(11) **EP 2 906 228 B1**
(45) Date de publication et mention de la délivrance du brevet: **07.06.2017**
(21) Numéro de dépôt: 13744712.4
(22) Date de dépôt: 08.07.2013
(51) Int. Cl.: A61K 33/00, A61P 25/00, A61K 9/00

(54) **UTILISATION D'ARGON POUR PREVENIR OU TRAITER LES CONSEQUENCES NEUROLOGIQUES D'UN CHOC SEPTIQUE**
VERWENDUNG VON ARGON ZUR VORBEUGUNG ODER BEHANDLUNG DER NEUROLOGISCHEN FOLGEN EINES SEPTISCHEN SCHOCKS
USE OF ARGON TO PREVENT OR TREAT THE NEUROLOGICAL CONSEQUENCES OF A SEPTIC SHOCK

(30) Priorité: 09.10.2012 FR 1259587
(43) Date de publication de la demande: 19.08.2015
(73) Titulaire: L'Air Liquide Société Anonyme pour l'Etude et l'Exploitation des Procédés Georges Claude, 75007 Paris (FR)
(72) Inventeur: LEMAIRE, Marc, F-75014 Paris (FR)
(74) Mandataire: Pittis, Olivier
(86) Numéro de dépôt international: PCT/FR2013/051621
(87) Numéro de publication internationale: WO 2014/057178

(56) Documents cités:
- WO-A2-2005/039600
- US-A1- 2011 086 107
- RYANG YU-MI ET AL: "Neuroprotective effects of argon in an in vivo model of transient middle cerebral artery occlusion in rats", CRITICAL CARE MEDICINE, LIPPINCOTT WILLIAMS & WILKINS, US, vol. 39, no. 6, 1 juin 2011 (2011-06-01), pages 1448-1453, XP009169625, ISSN: 1530-0293
- JAWAD N ET AL: "Neuroprotection (and lack of neuroprotection) afforded by a series of noble gases in an in vitro model of neuronal injury", NEUROSCIENCE LETTERS, LIMERICK, IE, vol. 460, no. 3, 4 septembre 2009 (2009-09-04), pages 232-236, XP026219307, ISSN: 0304-3940, DOI: 10.1016/J.NEULET.2009.05.069 [extrait le 2009-06-07]

## Description

L'invention porte sur l'utilisation d'argon gazeux pour fabriquer tout ou partie d'un médicament inhalable destiné à traiter et/ou à prévenir toute conséquence neurologique en rapport avec un choc septique.

Un choc septique ou infectieux est le résultat de la présence dans le sang de toxines bactériennes qui aboutit à une insuffisance de fonctionnement cardiaque, une oligurie ou diminution de la quantité des urines éliminées, et une acidose métabolique ou diminution du pH de l'organisme, l'ensemble survenant chez un sujet, c'est-à-dire un être humain, dont la température est supérieure à environ 38,9°C..

Un choc septique est principalement dû à des bactéries Gram (-), notamment les entérobactéries, tel que Escherichia Coli, Pseudomonas , Proteus, Serratia, Bacteroïdes Fragilis..., et dans ce cas la toxine est une endotoxine, ou des bactéries Gram (+), tel que les Pneumocoques, Streptocoque, Staphylocoques..., et dans ce cas la toxine est une exotoxine. Le choc septique peut aussi être causé par une levure, par exemple de type Candida, un virus ou un parasite, par exemple Plasmodium Falciparum.

Le choc septique est un problème majeur de santé publique puisqu'on dénombre environ 75 000 cas par an en France, et c'est l'une des principales causes de mortalité notamment en soins intensifs.

Ainsi, on évalue à 26% le nombre de décès en soins intensifs qui sont rapport avec un choc septique indépendamment de son étiologie primaire, selon Annane and al., Lancet 2005. En outre, près de 60% des patients ayant subi un choc septique mais ayant survécu présentent des déficits neurologiques sévères pouvant se confondre avec les signes d'une encéphalopathie or d'un délirium grave, comme rappelé par Eidelman and al, JAMA 1996. Par ailleurs, 30 à 50% des patients survivants présentent des séquelles neurologiques au long cours à titre de troubles neurocognitifs, selon Hiwashina, Ely and al., JAMA 2010.

L'épidémiologie du choc septique met en évidence une augmentation de cas, une augmentation de la mortalité et des séquelles associées de type troubles psycho-cognitifs associés à l'encéphalopathie septique, nécessitant de prévenir et de traiter ces lésions.

Ceci explique l'augmentation des travaux cliniques sur le sujet mais il n'existe aujourd'hui toujours aucun traitement spécifique de l'encéphalopathie et les dernières molécules testées se sont révélées inefficaces, voire même ont engrendré des effets secondaires néfastes pour les patients, comme le rappelle M.J. van Eijk and al., Lancet 2010.

La non-existence d'une thérapie efficace s'explique principalement par une méconnaissance des mécanismes physiopathologiques entrant en jeu lors des chocs septiques.

Récemment il a été suggéré que les altérations de la membrane encéphalo-hématologique résultantes de l'activation de sous-populations de cellules du cerveau, notamment les cellules microgliales et endothéliales, pourrait expliquer ces anomalies du fait d'une augmentation de la libération de médiateurs de l'inflammation et de substances neurotoxiques dans le cerveau, comme l'explique van Gool WA and al., Lancet 2010 ; et Iacobone and al., Crit Care Med 2009.

Il a été montré que certaines déficiences ou défaillance affectant le cerveau pouvaient être traitées à l'aide de gaz inhalés. Ainsi, le document EP-A-1158992 propose d'utiliser du xénon inhalé pour lutter contre les neuro-intoxications cérébrales, notamment ischémiques, qui se caractérisent par un dysfonctionnement cérébral d'un ou de plusieurs systèmes de neurotransmission.

Par ailleurs, le document EP-A-1541156 propose quant à lui d'utiliser de l'argon inhalé pour lutter contre lesdites neuro-intoxications cérébrales. Le potentiel neuroprotecteur de l'argon gazeux, administré seul ou en mélange avec du protoxyde d'azote, sur le développement et l'expression de la sensibilisation à la D-amphétamine a également été évalué.

Toutefois, les mécanismes d'action de l'argon sont encore mal connus et pourraient reposer sur une action agoniste des récepteurs GABA_{A}, notamment du site aux benzodiazépines, comme décrit par J. Abraini et al., Anesth Analg, 2003, 96:746-749.

Récemment, la capacité de neuroprotection de l'argon a été confirmée par Loetscher et al., Critical Care, 2009, 13 :R206 ;doi:10.1186/cc8214.

L'argon a été montré comme pouvant protéger des cellules cochléaires en culture isolée, c'est-à-dire les cellules dites chevelues issues de l'organe de Corti du rat, comme décrit par Yarin et al., Hear Res 2005, 201:1-9. Ces cellules sont assimilables aux neurones cérébraux, pour lesquels d'autres gaz, notamment le xénon, ont été montrés comme pouvant présenter un effet neuroprotecteur, comme rappelé par Ma et al., Br J Anaesth,2002, vol 98: 739-46.

En outre, le document EP-A-1651243 propose quant à lui d'utiliser des mélanges Xe/N₂O inhalé à cette fin, alors que le document DE-A-19991033704 propose des formulations liquides contenant du xénon dissout pour traiter les hypoxies et les ischémies cérébrales.

Comme on le voit, la plupart des traitements gazeux proposés visent à traiter des déficiences ou défaillance essentiellement cérébrales, c'est-à-dire qui affectent le cerveau, mais ces documents ne proposent pas de préserver le cerveau en cas de phénomènes inflammatoires responsables de lésions neurologiques à court et long terme, en particulier lorsqu'ils sont le résultat ou la conséquence d'un choc septique, en particulier un choc septique causé par la présence dans le sang d'une ou plusieurs endotoxines ou exotoxines..

Le problème est donc de proposer un traitement préventif et/ou curatif permettant de traiter et/ou de prévenir les conséquences neurologiques secondaires à un choc septique ou infectieux, c'est-à-dire les dégâts ou lésions neurologiques susceptibles d'être engendrés ou de résulter d'un choc septique.

La solution de l'invention est alors une composition gazeuse contenant de l'argon gazeux pour une utilisation par inhalation pour prévenir ou pour traiter la ou les conséquences neurologiques secondaires à un choc septique chez un patient.

En effet, dans le cadre de la présente invention, il a été mis en évidence qu'un gaz à base d'argon est capable de préserver le cerveau de phénomènes inflammatoires responsables de lésions neurologiques, à court et long terme, en cas de choc septique.

Selon le cas, la composition gazeuse ou médicament gazeux inhalable de l'invention peut comprendre l'une ou plusieurs des caractéristiques suivantes :
- la composition gazeuse contient une quantité efficace d'argon gazeux, c'est-à-dire au moins 10% en volume d'argon,
- la composition gazeuse contient entre 15 et 80% en volume d'argon.
- la composition gazeuse contient au moins 20% en volume d'argon.
- la composition gazeuse contient au moins 30% en volume d'argon.
- la composition gazeuse contient moins de 75% en volume d'argon.
- le choc septique résulte de la présence dans le sang d'une ou plusieurs toxines, notamment d'une ou plusieurs toxines d'origine bactérienne, virale, parasitaire ou issues d'une levure.
- le choc septique résulte de la présence dans le sang d'une ou plusieurs toxines d'origine bactérienne, en particulier des bactéries Gram négatif (G-), notamment des bactéries de type entérobactéries (Escherichia Coli, Pseudomonas Proteus, Serratia, Bacteroïdes Fragilis...). Dans ce cas, la toxine est une endotoxine.
- le choc septique résulte de la présence dans le sang d'une ou plusieurs toxines d'origine bactérienne, en particulier des bactéries Gram positif (G+), notamment des bactéries de type Pneumocoque, Streptocoque, Staphylocoque.... Dans ce cas, la toxine est une exotoxine.
- le choc septique résulte de la présence dans le sang d'une ou plusieurs toxines de levure, telle une Candida, ou d'origine virale ou un parasitaire, notamment un parasite de type Plasmodium Falciparum.
- le choc septique concerne un ou plusieurs autres organes que le cerveau, en particulier le foie, les reins, les poumons et/ou le coeur.
- la composition gazeuse contient en outre de l'oxygène, de préférence au moins 21% en volume d'oxygène.
- la composition gazeuse contient en outre un composé additionnel choisi dans le groupe formé par He, Ne, NO, CO et N₂.
- la composition gazeuse est exempte de xénon, c'est-à-dire qu'elle ne contient pas de xénon ou alors simplement à l'état de traces ou d'impuretés inévitables.
- l'argon est administré au patient, une ou plusieurs fois par jour pendant une durée d'inhalation de quelques minutes à une ou plusieurs heures.
- la composition gazeuse est formée d'un mélange gazeux contenant, en outre, de l'oxygène, de l'azote ou leurs mélanges, en particulier de l'air.
- la composition gazeuse est formée d'un mélange gazeux binaire constitué d'argon et d'oxygène pour le reste.
- la composition gazeuse est formée d'un mélange gazeux ternaire constitué d'argon, d'azote et d'oxygène.
- la composition gazeuse est un médicament gazeux prêt à l'emploi.
- la composition gazeuse est conditionnée dans une bouteille de gaz ayant une contenant (équivalent en eau) allant jusqu'à 50 litres, typiquement de l'ordre de 15 litres.
- la composition gazeuse est conditionnée dans une bouteille de gaz au sein de laquelle la composition gazeuse est à une pression inférieure ou égale à 350 bar.
- la composition gazeuse est conditionnée dans une bouteille de gaz équipée d'un robinet ou d'un robinet à détendeur intégré permettant de contrôler le débit et éventuellement la pression du gaz délivré.
- la composition gazeuse est conditionnée dans une bouteille de gaz en acier, en aluminium ou en matériaux composites.

Dit autrement, l'invention concerne également une utilisation d'argon gazeux pour fabriquer une composition médicamenteuse inhalable selon l'invention destinée à prévenir ou à traiter la ou les conséquences neurologiques secondaires à un choc septique.

D'une façon générale, lors du traitement, l'administration de la composition gazeuse au patient se fait par inhalation par exemple au moyen d'un ventilateur, d'un nébuliseur ou spontanément avec des bouteilles de gaz pré-conditionnées, raccordés à un masque facial ou nasal, ou des lunettes nasales.

La durée d'administration est fonction de la durée du choc septique, choisie au cas par cas affectant le patient considéré, par exemple l'argon peut être administré pendant une durée d'administration de quelques minutes à quelques dizaines de minutes, voire d'heures, par exemple moins d'une heure, à une fréquence pouvant atteindre une à plusieurs fois par jour et sur une durée totale de traitement de un ou plusieurs jours, semaines.

L'argon ou le mélange gazeux à base d'argon formant la composition gazeuse selon la présente invention est préférentiellement conditionné en bouteille de gaz sous pression ou sous forme liquide, par exemple dans une bouteille d'un à plusieurs litres (contenant en eau) et à une pression comprise entre 2 et 300 bar.

L'argon ou le mélange gazeux à base d'argon peut se présenter sous forme « prêt à l'emploi », par exemple en pré-mélange avec de l'oxygène, ou alors être mélangé sur site lors de son utilisation, notamment avec de l'oxygène et éventuellement un autre composé gazeux, par exemple de l'azote.

Dans le cadre de l'invention, le patient est un être humain, c'est-à-dire un homme ou une femme, y compris les enfants, les adolescents ou tout autre groupe d'individus, par exemple les nouveau-nés ou les sujets âgés.

### Exemple

Afin de montrer l'efficacité de l'argon dans le traitement ou la prévention des conséquences d'un choc septique, on a évalué les évènements précoces conduisant à une encéphalopathie dans le cadre d'un choc septique et des effets de l'argon gazeux sur les mécanismes en 3 étapes:
- Activation précoce des cellules microgliales *in situ* en réponse à un stimuli périphérique et responsable d'une libération de médiateurs de l'inflammation dans le tissu cérébral même quand la barrière hémato-méningée est encore étanche chez des souris CX3CR1^{GFP/+}_{.}
- Effets de l'argon dans un modèle in-vitro de cultures primaires de cellules microgliales de cerveaux sains et de cerveaux septiques.
- Preuve de concept sur une inflammation centrale et périphérique dans un modèle animal de choc septique.

Lors des phases initiales du sepsis, c'est-à-dire les 4 premiers jours, on a évalué, dans un modèle animal relevant et validé, la réaction du tissu nerveux en étudiant spécialement l'activation microgliale, tel que modifications morphologiques, modifications de l'état mitochondrial et de leurs biosynthèse *in situ* des médiateurs de l'inflammation (études protéomiques et génomiques).

On a étudié *in vitro*, l'effet de l'argon sur la modulation de l'activité microgliale.

Des cellules extraites de cerveaux de souris CX3CR1^{GFP/+} ont été exposées à de l'argon gazeux et la libération de médiateurs de l'inflammation et leur état mitochondrial ont été évalués.

On a ensuite étudié l'effet de l'argon dans une étude *in vivo* sur des animaux septiques et sur les contrôles. En particulier, ont été évalués le comportement clinique, la mortalité et les modifications neuropathologiques, tel que perte neuronale, activation microgliale et astrocytaire, neuroinflammation.

Le choc septique a été crée par ligature du caecum suivie d'une ouverture de ce dernier dans la cavité péritonéale chez des souris C57BL/6 et sur une souche génétiquement modifiée (knock-in mice CX3CR1^{GFP/+}). Cette souche de souris possède une fluorescence GFP dans les cellules microgliales. Son expression varie et est corrélée à l'intensité de l'activation des cellules microgliales.

Les effets de l'argon ont été évalués sur les modulations des modifications neuropathologiques et sur la neuro-inflammation.

Durant le choc septique, on a étudié:
- le taux de survie, la récupération post thérapeutique et notamment l'impact sur le comportement ;
- l'activation des cellules microgliales, le niveau d'apoptose (CD45, MHC antigène de classe 1, caspase activée 3, fragmentation du DNA), production des médiateurs de l'inflammation, NO et ROS ;
- l'impact neuropathologique (neurotoxicité, mort neuronale, prolifération astrocytaire)

Les résultats obtenus sont donnés ci-dessous.

### Etude in-vitro (cellules microligales) : modulation de l'activité cellulaire

Le Tableau I ci-dessous illustre la libération de médiateurs de l'inflammation et leur état mitochondrial suite à une mise en contact avec de l'air, de l'azote et, à titre comparatif, de l'argon selon la présente invention.

Une réponse favorable est désignée par un signe (-) correspondant à une preuve du ralentissement de l'activité, alors qu'une réponse défavorable par un signe (+) correspondant à un signal d'un accroissement de l'activité.

**Tableau I**

| | Cerveau sain | Cerveau avec sepsis |
|---|---|---|
| Groupe contrôle AIR (ref) | Niveau référence d'activité 0 | Niveau référence d'activité + |
| Groupe AZOTE | 0 | ++ |
| Groupe ARGON | ± | - |

### Etude in-vivo

Le Tableau II ci-dessous illustre le comportement clinique, la mortalité et les modifications neuropathologiques chez les animaux résultant d'une mise en contact avec de l'air, de l'azote et, à titre comparatif, de l'argon selon la présente invention.

Une réponse favorable est désignée par un signe (-), alors qu'une réponse défavorable par un signe (+).

**Tableau II**

| | Clinique | Mortalité | Modif Neuropath. |
|---|---|---|---|
| Groupe contrôle AIR | + | ++ | +++ |
| Groupe AZOTE | + | + | ++ |
| Groupe ARGON | - | - | - |

On constate, au vu des Tableaux I et II, que l'argon permet de lutter efficacement contre les chocs septiques chez les animaux testés.

### Bibliographie

- Annane D, Bellissant E, Cavaillon JM: Septicshock. Lancet 2005, 365:63-78
- Eidelman LA, Putterman D, Putterman C, Sprung CL: The spectrum of septicencephalopathy. Définition, etiologies, and mortalities.JAMA 1996, 275:470-473.
- Hiwashina, Ely and al.: Long-term Cognitive Impairment and Functional Disability Among Survivors of Severe Sepsis. JAMA 2010; 304(16):1787-1794
- Iacobone E, Bailly-Salin J, Polito A, Friedman D, Stevens RD, Sharshar T: Sepsisassociatedencephalopathy and its differential diagnosis. Crit Care Med 2009, 37:S331-336.
- Iborra and al, The functional organization of mitochondrial genomes in human cells. BMCBiology 2004, 2:9
- Jung S, Aliberti J, Graemmel P, Sunshine MJ, Kreutzberg GW, Sher A, Littman DR:Analysis of fractalkine receptor CX(3)CR1 function by targeted deletion and greenfluorescent protein reporter gene insertion. Mol Cell Biol 2000, 20:4106-4114.
- Kadoi and al.:An alteration in the gamma-aminobutyric acid receptor system in experimentally induced septic shock in rats. Crit Care Med 1996, 298-305
- Kakkar P, Singh BK. : Mitochondria : a hub of redox activities and cellular distress control.Mol Cell Biochem. 2007Nov ; 305(1-2):235-53
- MJ van Eijk and al., Effect of rivastigmine as an adjunct to usual care with haloperidol on duration of delirium and mortality in critically ill patients: a multicentre, double-blind placebo-controlled randomised trial. Lancet 2010 ;376: 1829-1837.
- San Jose MC, L.D., roxas AA, The clinical and biochemical features of adult patients with sepsis syndrome and encephalopathy. Phil J Microbiol Infect Dis 1999. 28(2): p. 46-52.
- Sharshar and al.: Brain lesions in septic shock, a MRI study. Intensive Care Medicine 2007: 33; 798-806
- Van Gool, W.A., D. van de Beek, and P. Eikelenboom, Systemic infection and delirium:when cytokines and acetylcholine collide. Lancet, 2010. 375(9716): p. 773-5.
- Weksler and al.: Blood-brain barrier-specific properties of a human adult brain endothelial cell line. FASEB J 2005 Nov;19(13):1872-4
- Westermann and al.: Mitochondrial fusion and fission in cell life and death. Nat Rev Mol CellBiol. 2010Dec ; 11 (12):872-84

## Revendications

1. Composition gazeuse contenant de l'argon gazeux pour une utilisation par inhalation pour prévenir ou traiter au moins une conséquence neurologique secondaire à un choc septique chez un patient.

2. Composition pour une utilisation selon la revendication précédente, **caractérisée en ce qu'**elle contient entre 15 et 80% en volume d'argon.

3. Composition pour une utilisation selon l'une des revendications précédentes, **caractérisée en ce qu'**elle contient au moins 20% en volume d'argon, de préférence au moins 30% en volume d'argon.

4. Composition pour une utilisation selon l'une des revendications précédentes, **caractérisée en ce qu'**elle contient moins de 75% en volume d'argon.

5. Composition pour une utilisation selon l'une des revendications précédentes, **caractérisée en ce que** le choc septique résulte de la présence dans le sang d'une ou plusieurs endo- ou exotoxines.

6. Composition pour une utilisation selon l'une des revendications précédentes, **caractérisée en ce que** le choc septique résulte de la présence dans le sang d'une ou plusieurs toxines d'origine bactérienne, virale, parasitaire ou issues d'une levure.

7. Composition pour une utilisation selon l'une des revendications précédentes, **caractérisée en ce que** le choc septique résulte de la présence dans le sang d'une ou plusieurs toxines d'origine bactérienne.

8. Composition pour une utilisation selon l'une des revendications précédentes, **caractérisée en ce que** au moins une conséquence neurologique est un phénomène inflammatoire susceptible d'engendrer une ou des lésions neurologiques.

9. Composition pour une utilisation selon l'une des revendications précédentes, **caractérisée en ce qu'**elle contient en outre de l'oxygène, de préférence au moins 21% en volume d'oxygène.

10. Composition pour une utilisation selon l'une des revendications précédentes, **caractérisée en ce qu'**elle contient en outre un composé additionnel choisi dans le groupe formé par N₂O, He, Ne, NO, CO, et N₂.

11. Composition pour une utilisation selon l'une des revendications précédentes, **caractérisée en ce qu'**elle est constituée d'argon et d'oxygène.

12. Composition pour une utilisation selon l'une des revendications précédentes, **caractérisée en ce qu'**elle est constituée d'argon, d'azote et d'oxygène.

13. Composition pour une utilisation selon l'une des revendications précédentes, **caractérisée en ce qu'**elle est exempte de xénon.

14. Composition gazeuse pour une utilisation selon l'une des revendications précédentes, **caractérisée en ce que** le choc septique affecte au moins un autre organe que le cerveau, ledit autre organe étant choisi parmi le foie, les reins, les poumons et le coeur.

## Patentansprüche

1. Gasförmige Zusammensetzung, enthaltend gasförmiges Argon für eine Verwendung durch Inhalierung, zur Vorbeugung oder Behandlung von mindestens einer sekundären neurologischen Folge eines septischen Schocks bei einem Patienten.

2. Zusammensetzung für eine Verwendung nach dem vorhergehenden Anspruch, **dadurch gekennzeichnet, dass** sie zwischen 15 und 80 Vol.% Argon enthält.

3. Zusammensetzung für eine Verwendung nach einem der vorhergehenden Ansprüche, **dadurch gekennzeichnet, dass** sie mindestens 20 Vol.% Argon, vorzugsweise mindestens 30 Vol.% Argon enthält.

4. Zusammensetzung für eine Verwendung nach einem der vorhergehenden Ansprüche, **dadurch gekennzeichnet, dass** sie mindestens 75 Vol.% Argon enthält.

5. Zusammensetzung für eine Verwendung nach einem der vorhergehenden Ansprüche, **dadurch gekennzeichnet, dass** sich der septische Schock aus dem Vorhandensein im Blut von einem oder mehreren Endo- oder Exotoxinen ergibt.

6. Zusammensetzung für eine Verwendung nach einem der vorhergehenden Ansprüche, **dadurch gekennzeichnet, dass** sich der septische Schock aus dem Vorhandensein im Blut von einem oder mehreren Toxinen bakteriellen, viralen, parasitären Ursprungs oder aus einer Hefe stammend ergibt.

7. Zusammensetzung für eine Verwendung nach einem der vorhergehenden Ansprüche, **dadurch gekennzeichnet, dass** sich der septische Schock aus dem Vorhandensein im Blut von einem oder mehreren Toxinen bakteriellen Ursprungs ergibt.

8. Zusammensetzung für eine Verwendung nach einem der vorhergehenden Ansprüche, **dadurch gekennzeichnet, dass** mindestens eine neurologische Folge ein Entzündungsphänomen ist, das eine oder mehrere neurologische Läsionen verursachen kann.

9. Zusammensetzung für eine Verwendung nach einem der vorhergehenden Ansprüche, **dadurch gekennzeichnet, dass** sie außerdem Sauerstoff, vorzugsweise mindestens 21 Vol.% Sauerstoff enthält.

10. Zusammensetzung für eine Verwendung nach einem der vorhergehenden Ansprüche, **dadurch gekennzeichnet, dass** sie außerdem eine zusätzliche Verbindung enthält, ausgewählt aus der Gruppe, bestehend aus N₂O, He, Ne, NO, CO und N₂.

11. Zusammensetzung für eine Verwendung nach einem der vorhergehenden Ansprüche, **dadurch gekennzeichnet, dass** sie aus Argon und Sauerstoff besteht.

12. Zusammensetzung für eine Verwendung nach einem der vorhergehenden Ansprüche, **dadurch gekennzeichnet, dass** sie aus Argon, Stickstoff und Sauerstoff besteht.

13. Zusammensetzung für eine Verwendung nach einem der vorhergehenden Ansprüche, **dadurch gekennzeichnet, dass** sie frei von Xenon ist.

14. Gasförmige Zusammensetzung für eine Verwendung nach einem der vorhergehenden Ansprüche, **dadurch gekennzeichnet, dass** der septische Schock mindestens ein weiteres Organ als das Gehirn betrifft, wobei das weitere Organ ausgewählt ist aus der Leber, den Nieren, den Lungen und dem Herzen.

## Claims

1. Gaseous composition containing gaseous argon for use by inhalation to prevent or treat at least one neurological side-effect of septic shock in a patient.

2. Composition for use according to the preceding claim, **characterised in that** it contains between 15 and 80% by volume of argon.

3. Composition for use according to one of the preceding claims, **characterised in that** it contains at least 20% by volume of argon, preferably at least 30% by volume of argon.

4. Composition for use according to one of the preceding claims, **characterised in that** it contains less than 75% by volume of argon.

5. Composition for use according to one of the preceding claims, **characterised in that** the septic shock is due to the presence of one or a plurality of endo- or exotoxins in the blood.

6. Composition for use according to one of the preceding claims, **characterised in that** the septic shock is due to the presence of one or a plurality of toxins of bacterial, viral, parasitic origin or derived from a yeast in the blood.

7. Composition for use according to one of the preceding claims, **characterised in that** the septic shock is due to the presence of one or a plurality of toxins of bacterial origin in the blood.

8. Composition for use according to one of the preceding claims, **characterised in that** at least one neurological side-effect is an inflammatory phenomenon liable to cause one or more neurological lesions.

9. Composition for use according to one of the preceding claims, **characterised in that** it further contains oxygen, preferably at least 21% by volume of oxygen.

10. Composition for use according to one of the preceding claims, **characterised in that** it further contains an additional compound chosen from the group formed by N₂O, He, Ne, NO, CO, and N₂.

11. Composition for use according to one of the preceding claims, **characterised in that** it consists of argon and oxygen.

12. Composition for use according to one of the preceding claims, **characterised in that** it consists of argon, nitrogen and oxygen.

13. Composition for use according to one of the preceding claims, **characterised in that** it is free from xenon.

14. Gaseous composition for use according to one of the preceding claims, **characterised in that** the septic shock affects at least one organ other than the brain, said other organ being chosen from the liver, kidneys, lungs and heart.
